# EUROPEAN PATENT APPLICATION

(11) **EP 4 582 096 A1**
(43) Date of publication of application: **09.07.2025**
(21) Application number: 23860903.6
(22) Date of filing: 31.08.2023
(51) Int. Cl.: A61K 38/17, A61P 37/00, A61P 13/12, A23L 33/17

(54) **COMPOSITION FOR PREVENTING OR TREATING LUPUS, COMPRISING RECOMBINANT STABILIZED GALECTIN 9 PROTEIN**

(30) Priority: 31.08.2022 KR 20220109953
(71) Applicant: Gbiologics Inc., Seongnam-si, Gyeonggi-do 13487 (KR)
(72) Inventor: SONG, David, Seongnam-si Gyeonggi-do 13487 (KR)
(74) Representative: Ström & Gulliksson AB
(86) International application number: PCT/KR2023/012952
(87) International publication number: WO 2024/049227

(57) **Abstract**

The present invention relates to a composition for preventing or treating lupus or glomerulonephritis comprising a recombinant stabilized Galectin-9 protein. Specifically, the recombinant stabilized Galectin-9 protein of the present invention has been confirmed to exhibit safety in a systemic lupus erythematosus (SLE) animal model, reduce skin lesions, lymphadenopathy, and proteinuria caused by lupus, ameliorate lupus nephritis and glomerulonephritis, and decrease the concentration of anti-dsDNA antibodies in plasma. Accordingly, the recombinant stabilized Galectin-9 protein of the present invention can be effectively used as an active ingredient in a composition for preventing or treating lupus or glomerulonephritis.

## Description

### [Technical Field]

The present invention relates to a recombinant stabilized galectin-9 protein and uses thereof, and more specifically, to a composition for preventing or treating lupus or glomerulonephritis, comprising the above protein as an active ingredient.

### [Background Art]

It has been discovered that animal lectins specifically recognizing glycans with a β-galactoside structure exist in living organisms, and to date, at least 14 types of genes have been identified. Based on their structure, galectins are classified into prototype, chimera, and tandem repeat types.

Galectin-9, a type of tandem repeat galectin, consists of two carbohydrate recognition domains (CRDs) and a linker peptide region connecting them. Through this linker peptide region, the N-terminal carbohydrate recognition domain (NCRD) and the C-terminal carbohydrate recognition domain (CCRD) are linked. To date, galectin-9 has been reported to exhibit various biological activities. With respect to T cells, galectin-9 binds to Tim-3, induces apoptosis of Tim-3-positive Th1 cells, and suppresses excessive Th1 responses, thereby inhibiting autoimmune inflammation.

In order to utilize galectin-9 as a therapeutic agent, ongoing research has focused on addressing the issues of (1) protease sensitivity, (2) low solubility, and (3) low yield. Efforts include modifying the linker peptide of galectin-9 to enhance protease resistance, such as by generating a galectin-9 variant (G9Null) in which the linker peptide is cleaved.

Meanwhile, lupus is an autoimmune disease associated with antibodies that attack connective tissues and is linked to the production of circulating immune complexes, such as antinuclear antibodies, and the activation of the complement system. In general, the term "lupus" primarily refers to systemic lupus erythematosus (SLE). This disease is systemic in nature, affecting all organ systems and potentially causing severe tissue damage. Patients with lupus may produce autoantibodies with specificities against DNA, Ro, and platelets, leading to the development of conditions such as glomerulonephritis, arthritis, serositis, complete heart block in neonates, or hematological abnormalities.

If left untreated, lupus can progress from attacking the skin and joints to affecting internal organs, such as the lungs, heart, and kidneys, and may become life-threatening. Among these, kidney disease is of the greatest concern. Renal damage, measured by the amount of proteinuria in urine, is one of the acute injury sites associated with the pathogenesis of lupus and accounts for more than 50% of lupus-related mortality and morbidity.

Currently, there is no definitive cure for lupus. From a practical perspective, physicians generally administer various immunosuppressive agents, such as high-dose corticosteroids, prednisone, azathioprine, or cyclophosphamide. However, a significant issue with many of these drugs is that they exhibit potentially harmful side effects in treated patients.

Accordingly, the present inventors endeavored to develop a safe therapeutic agent for lupus and, as a result, successfully produced a recombinant stabilized galectin-9 protein in which the amino acids of the C-terminal carbohydrate recognition domain (CCRD) and the linker peptide of conventional wild-type galectin-9 are deleted and substituted. The safety of the recombinant stabilized galectin-9 protein was confirmed in an in vivo animal model of systemic lupus erythematosus. Furthermore, in the *in vivo* animal model of systemic lupus erythematosus, the recombinant stabilized galectin-9 protein was found to reduce lupus-induced skin lesions, lymphadenopathy, and proteinuria, alleviate lupus nephritis and glomerulonephritis, and decrease plasma anti-dsDNA antibody levels. These findings demonstrate that the recombinant stabilized galectin-9 protein can be effectively used as an active ingredient in a composition for preventing or treating lupus or glomerulonephritis, leading to the present application.

### [Prior Art Documents]

### [Patent Documents]

KR Patent Publication No. 10-2019-0060550

### [Non-Patent literature]

Nishi, N et al. Development of highly stable galectins: truncation of the linker peptide confers protease-resistance on tandem-repeat type galectins. FEBS Lett. 2005 Apr 11;579(10):2058-64
Wang, Y, Xiao, S., Xia, Y. et al. The Therapeutic Strategies for SLE by Targeting Anti-dsDNA Antibodies. Clinic Rev Allerg Immunol(2021). https://doi.org/10.1007/s12016-021-08898-7

### [Detailed Description of the Invention]

### [Technical Problem]

The object of the present invention is to provide a composition for preventing or treating lupus or glomerulonephritis comprising a recombinant stabilized galectin-9 protein.

### [Solution to Problem]

To achieve the object of the present invention, the present invention provides: a pharmaceutical composition for preventing or treating lupus or glomerulonephritis, comprising a recombinant stabilized Galectin-9 protein having the amino acid sequence set forth in Sequence No. 1 or a polynucleotide encoding the same as an active ingredient; a method for preventing or treating lupus or glomerulonephritis, comprising administering to a subject a pharmaceutical composition comprising a recombinant stabilized Galectin-9 protein having the amino acid sequence set forth in Sequence No. 1 or a polynucleotide encoding the same; a recombinant stabilized Galectin-9 protein having the amino acid sequence set forth in Sequence No. 1 or a polynucleotide encoding the same for use in preventing or treating lupus or glomerulonephritis; and the use of a recombinant stabilized Galectin-9 protein having the amino acid sequence set forth in Sequence No. 1 or a polynucleotide encoding the same for the manufacture of a composition for preventing or treating lupus or glomerulonephritis.

Furthermore, the present invention provides: a health functional food composition for preventing or ameliorating lupus or glomerulonephritis, comprising a recombinant stabilized Galectin-9 protein having the amino acid sequence set forth in Sequence No. 1 or a polynucleotide encoding the same as an active ingredient; a recombinant stabilized Galectin-9 protein having the amino acid sequence set forth in Sequence No. 1 or a polynucleotide encoding the same for use in preventing or ameliorating lupus or glomerulonephritis; and the use of a recombinant stabilized Galectin-9 protein having the amino acid sequence set forth in Sequence No. 1 or a polynucleotide encoding the same for the manufacture of a health functional food composition for preventing or ameliorating lupus or glomerulonephritis.

### [Advantageous Effects of Invention]

The recombinant stabilized Galectin-9 protein of the present invention has been confirmed to exhibit safety in a systemic lupus erythematosus (SLE) animal model, reduce skin lesions, lymphadenopathy, and proteinuria caused by lupus, ameliorate lupus nephritis and glomerulonephritis, and decrease the concentration of anti-dsDNA antibodies in the blood. Accordingly, the recombinant stabilized Galectin-9 protein of the present invention can be effectively used as an active ingredient in a composition for preventing or treating lupus or glomerulonephritis.

### [Brief Description of Drawings]

Fig. 1 is a diagram showing the changes in body weight in an *in vivo* model of systemic lupus erythematosus (SLE) administered with the recombinant stabilized Galectin-9 protein (sGal-9) of the present invention.
Fig. 2 is a diagram illustrating the severity of skin lesions scored in an *in vivo* model of systemic lupus erythematosus (SLE) administered with the sGal-9 of the present invention.
Fig. 3 is a diagram illustrating the changes in lymphadenopathy scored in an *in vivo* model of systemic lupus erythematosus (SLE) administered with the sGal-9 of the present invention.
Fig. 4 is a diagram illustrating the severity of proteinuria scored in an *in vivo* model of systemic lupus erythematosus (SLE) administered with the sGal-9 of the present invention.
Fig. 5 is a diagram showing the changes in spleen weight in an *in vivo* model of systemic lupus erythematosus (SLE) administered with the sGal-9 of the present invention.
Fig. 6 is a diagram showing the histological changes in the kidney in an *in vivo* model of systemic lupus erythematosus (SLE) administered with the sGal-9 of the present invention.
Fig. 7 is a diagram showing the degree of IgG deposition in the kidney in an *in vivo* model of systemic lupus erythematosus (SLE) administered with the sGal-9 of the present invention.
Fig. 8 is a diagram showing the concentration of anti-dsDNA antibodies in plasma in an *in vivo* model of systemic lupus erythematosus (SLE) administered with the sGal-9 of the present invention.

### [Best Mode for Carrying Out the Invention]

Hereinafter, embodiments of the present invention will be described in detail to enable those of ordinary skill in the art to readily practice the invention. The embodiments of the present invention are provided to more fully illustrate the invention to those of ordinary skill in the art. Accordingly, the embodiments of the present invention may be modified in various ways, and the scope of the present invention is not limited to the embodiments described below.

Throughout the specification of the present invention, when a certain part is described as "comprising" a certain component, it means that other components may be additionally included unless explicitly stated otherwise, rather than excluding other components.

The present invention provides: a pharmaceutical composition for preventing or treating lupus or glomerulonephritis, comprising a recombinant stabilized Galectin-9 protein having the amino acid sequence set forth in Sequence No. 1 or a polynucleotide encoding the same as an active ingredient; a method for preventing or treating lupus or glomerulonephritis, comprising administering to a subject a pharmaceutical composition comprising a recombinant stabilized Galectin-9 protein having the amino acid sequence set forth in Sequence No. 1 or a polynucleotide encoding the same; a recombinant stabilized Galectin-9 protein having the amino acid sequence set forth in Sequence No. 1 or a polynucleotide encoding the same for use in preventing or treating lupus or glomerulonephritis; and the use of a recombinant stabilized Galectin-9 protein having the amino acid sequence set forth in Sequence No. 1 or a polynucleotide encoding the same for the manufacture of a composition for preventing or treating lupus or glomerulonephritis.

In the present invention, the recombinant stabilized Galectin-9 protein may have an effect of preventing or treating lupus or glomerulonephritis without side effects such as changes in body weight.

As used in the present invention, the term "prevention" refers to any action that suppresses or delays the onset of a disease through the administration of the composition.

As used in the present invention, the term "treatment" refers to any action in which the symptoms of the disease are improved or beneficially altered through the administration of the composition.

In the present invention, the recombinant stabilized Galectin-9 protein is a protein that retains the glycan-recognition activity of wild-type Galectin-9 while possessing a more stable molecular structure against proteases.

Specifically, the recombinant stabilized Galectin-9 protein is a recombinant protein engineered by modifying the linker region connecting the two carbohydrate recognition domains (CRDs) and the C-terminal carbohydrate recognition domain (CCRD) of wild-type Galectin-9, which has an NCRD-linker-CCRD structure. More specifically, the recombinant stabilized Galectin-9 protein is constructed such that the peptide sequence of the linker region is entirely deleted, and in the CCRD (Sequence No. 2), the amino acid sequence at positions 1 to 10 (Sequence No. 3) is deleted, and the alanine (A) at position 13 is substituted with proline (P), resulting in an amino acid sequence set forth in Sequence No. 1. The protein may include an amino acid sequence having at least 75% sequence homology, preferably at least 80%, more preferably at least 90%, and most preferably at least 95% sequence homology to the amino acid sequence set forth in Sequence No. 1. Additionally, the recombinant stabilized Galectin-9 protein may further include a targeting sequence, a tag, labeled residues, or amino acid sequences designed for specific purposes such as increasing half-life or peptide stability.

Additionally, the recombinant stabilized Galectin-9 protein may include the deletion of the first amino acid residue from the N-terminus of the amino acid sequence set forth in Sequence No. 1 and, specifically, may comprise the amino acid sequence set forth in Sequence No. 4.

As used in the present invention, the term "polynucleotide" refers to a polymer of nucleotides that functions to transmit genetic information. For the purposes of the present invention, the polynucleotide encodes the recombinant protein of Sequence No. 1 and may include a sequence having at least 75% sequence homology, preferably at least 85%, more preferably at least 90%, and most preferably at least 95% sequence homology to the polynucleotide sequence encoding the recombinant protein.

The term "homology" as used in the present invention is intended to indicate the degree of similarity to a wild-type amino acid sequence or polynucleotide sequence, and such homology comparison may be performed using comparison programs widely known in the art. The homology between two or more sequences can be calculated as a percentage (%).

The term "lupus" as used in the present invention refers to an antibody-related autoimmune disease that attacks connective tissues and includes chronic inflammatory autoimmune diseases characterized by the presence of autoantibodies, rash, oral ulcers, serositis, neurological disorders, low blood cell counts, joint pain, and swelling. Unless otherwise specified, the term "lupus" in the present invention has the conventional meaning as used in the technical field to which the present invention pertains. In the present invention, lupus may include, but is not limited to, various additional forms of lupus, such as systemic lupus erythematosus (SLE), systemic lupus, discoid lupus, drug-induced lupus, or neonatal lupus. Additionally, chronic nephritis, such as lupus nephritis or glomerulonephritis, may be caused by lupus.

The systemic lupus erythematosus (SLE) referred to herein is also known as systemic lupus or systemic erythematosus and has the conventional meaning as used in the technical field to which the present invention pertains. SLE is a multiorgan autoimmune disease in which antinuclear antibodies, including anti-dsDNA antibodies, are produced, leading to the formation of antigen-antibody immune complexes that deposit in small blood vessels. This deposition can cause inflammation and damage to multiple organs, including the basement membrane of the skin and kidneys.

The discoid lupus referred to herein is a disease localized to the skin. It typically manifests as rashes on areas such as the face, neck, and limbs. While the affected area is not as extensive as in systemic lupus, the symptoms are generally severe, and if not properly treated, they may result in hyperpigmentation or scarring. Approximately 10% of patients with discoid lupus may later develop systemic lupus, and 10-20% of systemic lupus patients may exhibit discoid skin lesions.

The drug-induced lupus referred to herein occurs after the administration of certain medications and presents symptoms similar to those observed in systemic lupus erythematosus, such as fever, rash, and joint pain. The most commonly associated drugs include hydralazine, a medication used for the treatment of hypertension, and procainamide, an antiarrhythmic agent.

In the present invention, the pharmaceutical composition can prevent or treat lupus by reducing skin lesions, lymphadenopathy, or proteinuria caused by lupus, as well as by decreasing the concentration of anti-dsDNA antibodies in the blood.

Additionally, the pharmaceutical composition can prevent or treat lupus nephritis or glomerulonephritis by inhibiting mesangial proliferation, reducing the infiltration of inflammatory cells in the glomeruli, and decreasing the renal deposition of immune complexes.

In a specific embodiment of the present invention, the inventors prepared a recombinant stabilized galectin-9 protein (sGal-9), in which the C-terminal carbohydrate recognition domain (CCRD) and amino acids of the linker peptide in the two glycan recognition sites of the wild-type galectin-9 were deleted and substituted.

Additionally, the inventors confirmed that repeated administration of the sGal-9 in an *in vivo* systemic lupus erythematosus model did not result in any changes in body weight.

Additionally, the inventors confirmed that in an *in vivo* systemic lupus erythematosus model, the sGal-9 reduced skin lesions, suppressed lymphadenopathy, and decreased the amount of proteinuria, thereby alleviating the clinical symptoms caused by lupus.

Additionally, the inventors confirmed that in an *in vivo* systemic lupus erythematosus model, the sGal-9 exhibited a therapeutic effect on lupus nephritis and glomerulonephritis by inhibiting mesangial proliferation and reducing the infiltration of inflammatory cells in and around the glomeruli as well as the renal deposition of immune complexes.

Furthermore, the inventors confirmed that in an *in vivo* systemic lupus erythematosus model, the sGal-9 exhibited the effect of reducing the concentration of anti-dsDNA antibodies in plasma.

Accordingly, the inventors confirmed that the recombinant stabilized galectin-9 protein according to the present invention exhibited safety in an animal model of systemic lupus erythematosus, alleviated clinical symptoms caused by lupus, lupus nephritis, and glomerulonephritis, and reduced the concentration of anti-dsDNA antibodies in the blood. Therefore, the recombinant stabilized galectin-9 protein or a polynucleotide encoding the same according to the present invention can be advantageously used as an active ingredient in a pharmaceutical composition for the prevention or treatment of lupus or glomerulonephritis.

In the present invention, the recombinant stabilized galectin-9 protein or a polynucleotide encoding the same may be carried by a pharmaceutically acceptable carrier such as a colloidal suspension, powder, saline, lipid, liposome, microsphere, or nano-spherical particle. These may form a complex with or be associated with a delivery vehicle and can be delivered in vivo using carrier systems known in the art, including lipids, liposomes, microparticles, gold, nanoparticles, polymers, condensation agents, polysaccharides, polyamino acids, dendrimers, saponins, absorption enhancers, or fatty acids.

Additionally, the pharmaceutically acceptable carrier may include, but is not limited to, lactose, dextrose, sucrose, sorbitol, mannitol, starch, acacia, gum, calcium phosphate, alginate, gelatin, calcium silicate, microcrystalline cellulose, polyvinylpyrrolidone, cellulose, water, syrup, methylcellulose, methyl hydroxybenzoate, propyl hydroxybenzoate, talc, magnesium stearate, and mineral oil, which are commonly used in formulation. Furthermore, in addition to the aforementioned components, the composition may further include lubricants, humectants, sweeteners, flavoring agents, emulsifiers, suspending agents, and preservatives.

In the present invention, the pharmaceutical composition of the present invention may be administered orally or parenterally (e.g., intramuscularly, intravenously, intraperitoneally, subcutaneously, intradermally, or topically) depending on the intended method. The dosage may vary depending on the condition and body weight of the patient, the severity of the disease, the dosage form, the route of administration, and the timing of administration, but it can be appropriately selected by those skilled in the art.

The pharmaceutical composition of the present invention is administered in a pharmaceutically effective amount.

The term "pharmaceutically effective amount" as used in the present invention refers to an amount sufficient to treat a disease with a reasonable benefit/risk ratio applicable to medical treatment. The effective dosage level may be determined based on factors such as the type and severity of the disease in the patient, the activity of the drug, the patient's sensitivity to the drug, the timing and route of administration, the rate of excretion, the duration of treatment, concurrently used medications, and other factors well known in the medical field. The pharmaceutical composition according to the present invention may be administered as a monotherapy or in combination with surgery, hormone therapy, drug therapy, or biological response modifiers. It may be administered simultaneously, separately, or sequentially with the aforementioned therapies and may be given as a single or multiple doses. Considering all these factors, it is important to administer the minimum amount necessary to achieve the maximum effect without side effects, which can be readily determined by those skilled in the art.

Specifically, the effective amount of the pharmaceutical composition of the present invention may vary depending on the age, sex, condition, and body weight of the patient, the absorption, inactivation rate, and excretion rate of the active ingredient in the body, the type of disease, and the co-administered drugs, and may be increased or decreased according to the route of administration, severity of obesity, sex, body weight, and age.

In the present invention, the pharmaceutical composition may be formulated into a dosage form selected from the group consisting of tablets, capsules, injections, troches, powders, granules, solutions, suspensions, internal liquids, emulsions, syrups, suppositories, vaginal tablets, and pills, but is not limited thereto and may be formulated into an appropriate dosage form as needed. In addition, when formulating the composition, it is generally prepared using diluents or excipients such as fillers, extenders, binders, wetting agents, disintegrants, and surfactants, which are commonly used in the art.

Solid preparations for oral administration include tablets, pills, powders, granules, capsules, and troches, and such solid preparations are prepared by mixing at least one excipient, such as starch, calcium carbonate, sucrose, lactose, or gelatin, with one or more recombinant proteins of the present invention. In addition to simple excipients, lubricants such as magnesium stearate and talc may also be used. Liquid preparations for oral administration include suspensions, internal liquids, emulsions, and syrups, which may contain commonly used simple diluents such as water and liquid paraffin, as well as various excipients such as wetting agents, sweeteners, flavoring agents, and preservatives.

Preparations for parenteral administration include sterile aqueous solutions, non-aqueous solvents, suspension solvents, emulsions, lyophilized formulations, and suppositories.

Non-aqueous solvents and suspension solvents may include propylene glycol, polyethylene glycol, vegetable oils such as olive oil, and injectable esters such as ethyl oleate. Bases for suppositories may include witepsol, macrogol, tween 61, cacao butter, laurin butter, glycerol, and gelatin.

Additionally, the present invention provides a health functional food composition for preventing or ameliorating lupus or glomerulonephritis, comprising as an active ingredient a recombinant stabilized galectin-9 protein having the amino acid sequence set forth in Sequence No. 1 or a polynucleotide encoding the same; a recombinant stabilized galectin-9 protein having the amino acid sequence set forth in Sequence No. 1 or a polynucleotide encoding the same for use in preventing or ameliorating lupus or glomerulonephritis; and the use of a recombinant stabilized galectin-9 protein having the amino acid sequence set forth in Sequence No. 1 or a polynucleotide encoding the same for the manufacture of a health functional food composition for preventing or ameliorating lupus or glomerulonephritis.

In the present invention, the descriptions of the recombinant stabilized galectin-9 protein and lupus are the same as those provided above, and thus, the detailed description thereof is incorporated herein by reference.

As used in the present invention, the term "amelioration" refers to any action that at least reduces a parameter related to the treated condition, such as the severity of symptoms. In this regard, the health functional food composition may be used either before the onset of the disease or after its onset, either concurrently with or separately from a therapeutic agent for the treatment of the disease, for the prevention or amelioration of the disease.

Meanwhile, in the present invention, it was confirmed that the recombinant stabilized galectin-9 protein according to the present invention exhibits safety in a systemic lupus erythematosus animal model, alleviates clinical symptoms caused by lupus, lupus nephritis, and glomerulonephritis, and reduces the concentration of anti-dsDNA antibodies in the blood. Accordingly, the recombinant stabilized galectin-9 protein or a polynucleotide encoding the same according to the present invention can be usefully employed as an active ingredient in a health functional food composition for preventing or ameliorating lupus.

In the health functional food of the present invention, the active ingredient may be directly added to food or used in combination with other foods or food ingredients and may be appropriately utilized according to conventional methods. The amount of the active ingredient to be mixed may be suitably determined depending on its intended use (prevention or amelioration). Generally, in the production of food or beverages, the health functional food of the present invention may be added in an amount of preferably 15 wt% or less, more preferably 10 wt% or less, based on the raw materials. However, in cases of long-term consumption for health and hygiene purposes or for health regulation purposes, the amount may be lower than the above range.

The health functional food of the present invention, in addition to containing the above-described active ingredient, may contain other components as essential ingredients without particular limitation. For example, it may include various flavoring agents or natural carbohydrates as additional components, similar to conventional beverages. Examples of the aforementioned natural carbohydrates include conventional sugars, such as monosaccharides (e.g., glucose and fructose); disaccharides (e.g., maltose and sucrose); and polysaccharides (e.g., dextrin and cyclodextrin), as well as sugar alcohols such as xylitol, sorbitol, and erythritol. In addition to the above, natural flavoring agents such as thaumatin and stevia extracts (e.g., rebaudioside A, glycyrrhizin) and synthetic flavoring agents such as saccharin and aspartame may be advantageously used. The proportion of the natural carbohydrates may be appropriately determined at the discretion of those skilled in the art.

In addition, the health functional food of the present invention may contain various nutrients, vitamins, minerals (electrolytes), synthetic and natural flavoring agents, colorants, and texture enhancers (such as cheese and chocolate), as well as pectic acid and its salts, alginic acid and its salts, organic acids, protective colloid thickeners, pH adjusters, stabilizers, preservatives, glycerin, alcohol, and carbonating agents used in carbonated beverages. These components may be used independently or in combination, and the proportion of such additives may also be appropriately selected by those skilled in the art.

The present invention is described in further detail below through Preparation Examples and Experimental Examples. However, the following Preparation Examples and Experimental Examples are provided merely to aid in understanding the present invention and are not intended to limit the scope of the present invention.

### <Preparation Example 1> Production of Recombinant Stabilized Galectin-9 Protein (sGal-9)

An expression vector containing a gene encoding a recombinant stabilized galectin-9 protein having the amino acid sequence set forth in Sequence No. 1 was constructed, and the expression vector was introduced into *Escherichia coli* (*E. coli*) using the heat shock method. Expression of the recombinant protein was induced by culturing *E*. *coli* in LB medium containing 50 µg/mL kanamycin and adding arabinose when the absorbance at 600 nm reached 0.7. Thereafter, the cells in which recombinant protein expression was induced were lysed and filtered, and the target protein was captured using cation exchange chromatography and affinity columns. As a result, recombinant stabilized galectin-9 protein with high purity was obtained in high yield.

### <Experimental Example 1> Safety Evaluation of sGal-9 in an In Vivo Model of Systemic Lupus Erythematosus (SLE)

To evaluate the efficacy of sGal-9 in lupus, the safety of sGal-9 was assessed after administering the sGal-9 of the present invention to an *in vivo* model of systemic lupus erythematosus (SLE).

### <1-1> Establishment of an In Vivo Model of Systemic Lupus Erythematosus and Administration of sGal-9

To evaluate the efficacy of sGal-9 in lupus, an *in vivo* model of systemic lupus erythematosus (SLE) was established, and the sGal-9 of the present invention was administered.

Specifically, MRL/FAS^{lpr} mice are generally known to develop disease due to the MRL-*lpr* or *lpr* mutation. These mice are homozygous for the Fas*lpr* spontaneous lymphoproliferation mutation and exhibit systemic autoimmunity, extensive lymphadenopathy associated with abnormal T-cell proliferation, arthritis, and immune complex-mediated glomerulonephritis. In these mice, the level of circulating immune complexes significantly increases from approximately 3 months of age, and lymph node proliferation is approximately 75 times greater in MRL/Mp-Fas*lpr*/Fas*lpr* mice compared to control lymph node weight. Additionally, extensive renal pathological differences appear between 4 and 7 months of age, making this model useful for evaluating therapeutic effects on systemic lupus erythematosus (SLE). Accordingly, 11-week-old female MRL/FAS^{lpr} mice were purchased from Central Lab. Animal, Inc. and used as an in vivo model for systemic lupus erythematosus. The breeding and experiments using MRL/FAS^{lpr} mice were conducted in a specific pathogen-free (SPF) animal facility, where the temperature was maintained at 21-23°C and the relative humidity was maintained at 40-45%. Experimental rodents were provided ad libitum access to rodent chow from Envigo, Inc.

The 11-week-old female MRL/FAS^{lpr} mice were randomly assigned into four groups as shown in [Table 1]: a vehicle-treated negative control group (Vehicle), a mycophenolate-treated positive control group (Mycophenolate), an sGal-9 2 mg/kg treatment group (sGal-9 2 mg/kg), and an sGal-9 4 mg/kg treatment group (sGal-9 4 mg/kg). The negative control group received a subcutaneous injection of vehicle once per week. The positive control group received an oral administration of mycophenolate (Roche), a drug used for the treatment of lupus nephritis, at a dose of 60 mg/kg once daily. The sGal-9 2 mg/kg treatment group and the sGal-9 4 mg/kg treatment group received subcutaneous injections of sGal-9, prepared as described in <Preparation Example 1>, at doses of 2 mg/kg and 4 mg/kg, respectively, once per week. Each test substance was administered for a total of nine weeks.

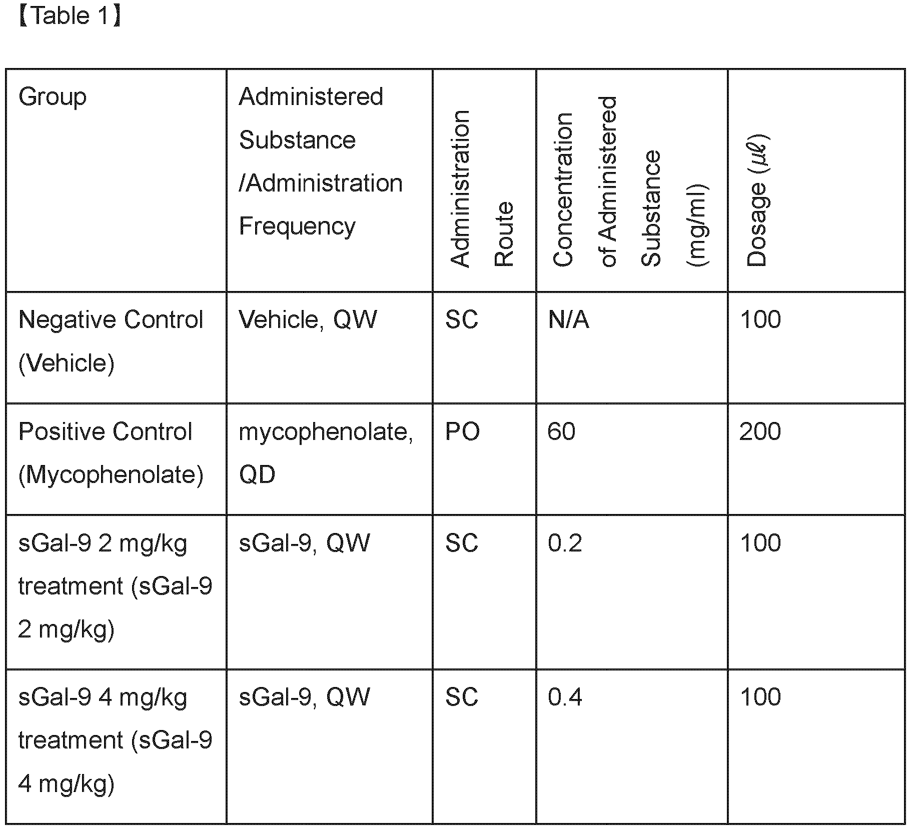

In addition, statistical analysis between the negative control group and the test groups or between the two test groups was performed using SPSS based on blinded evaluation data for each assessment parameter. For comparisons between two groups, Student's *t*-test or the Mann-Whitney *U* test was used. To compare differences among treatment groups at multiple time points, repeated measures ANOVA with Tukey's post-hoc test was performed. A significance level was set at a *p*-value of 0.05 or less.

### <1-2> Body Weight Assessment in an In Vivo Model of Systemic Lupus Erythematosus

To evaluate the safety of the sGal-9 of the present invention in an in vivo model of systemic lupus erythematosus, the body weight of the experimental animals from <1-1> was measured.

Specifically, body weight was measured during the 9-week repeated administration of the test substances according to the method described in <1-1>.

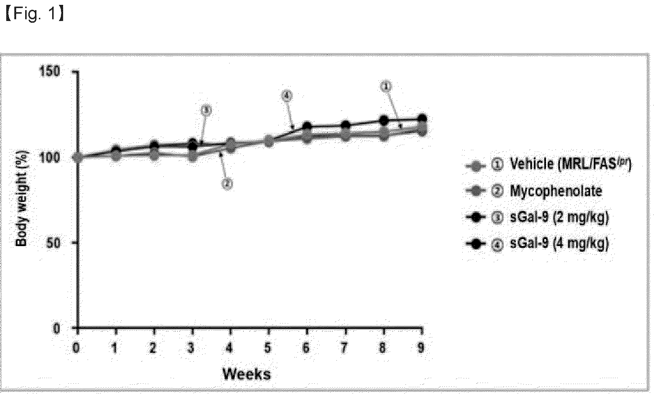

As a result, as shown in Fig. 1, it was confirmed that there were no statistically significant changes in body weight in the negative control group (Vehicle), the positive control group (Mycophenolate), the sGal-9 2 mg/kg treatment group (sGal-9 2 mg/kg), and the sGal-9 4 mg/kg treatment group (sGal-9 4 mg/kg).

### <Experimental Example 2> Evaluation of Clinical Indicators After Administration of sGal-9 in an In Vivo Model of Systemic Lupus Erythematosus

To evaluate the efficacy of the sGal-9 of the present invention in an in vivo model of systemic lupus erythematosus, clinical indicators were assessed in the experimental animals from <1-1>.

Specifically, the occurrence and severity of lupus were regularly observed from group allocation to the end of the experiment in the experimental animals from <1-1> and evaluated according to the criteria shown in [Table 2].

**[Table 2]**

| Clinical Evaluation Parameters | | | |
|---|---|---|---|
| Score | Proteinuria score | Skin score | Lymphadenopat hy score |
| 0 | Negative | No Symptoms (None) | No Symptoms (None) |
| 1 | (+)/(0.3 g/L)/(30 mg/dL) | 1 or 2 small lesions (2-4mm) in face, minimal hair loss with redness | Small, one site |
| 2 | (++)/(1 g/L)/(100 mg/dL) | Lesion larger than 1 but total area <5 cm² in face and ears, redness, scabbing and hair loss | Moderate, two different sites |
| 3 | (+++)/(3 g/L)/(300 mg/dL) | Lesion with total area >5 cm² <1 cm² face and ears and backs, ulcerations with an extensive area of involvement | Large, three different sites |
| 4 | (++++)/(≥20 g/L)/(≥2000 mg/dL) | Lesion with total area >1 cm² face and ears and backs, ulcerations with an extensive area of involvement | - |

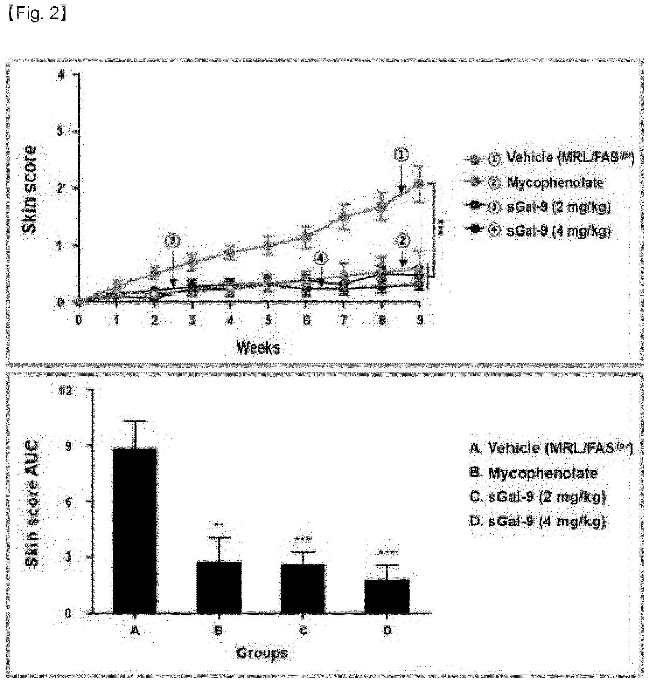

Additionally, after the completion of the experiment, the experimental animals from <1-1> were sacrificed, and the spleens were extracted for weight measurement. As a result, as shown in Fig. 2, it was confirmed that skin lesions were reduced in the positive control group (Mycophenolate) and the sGal-9 treatment groups (sGal-9 2 mg/kg and sGal-9 4 mg/kg) compared to the negative control group (Vehicle). Notably, the reduction in skin lesions was more pronounced in the sGal-9 treatment groups (sGal-9 2 mg/kg and sGal-9 4 mg/kg) compared to the positive control group (Mycophenolate).

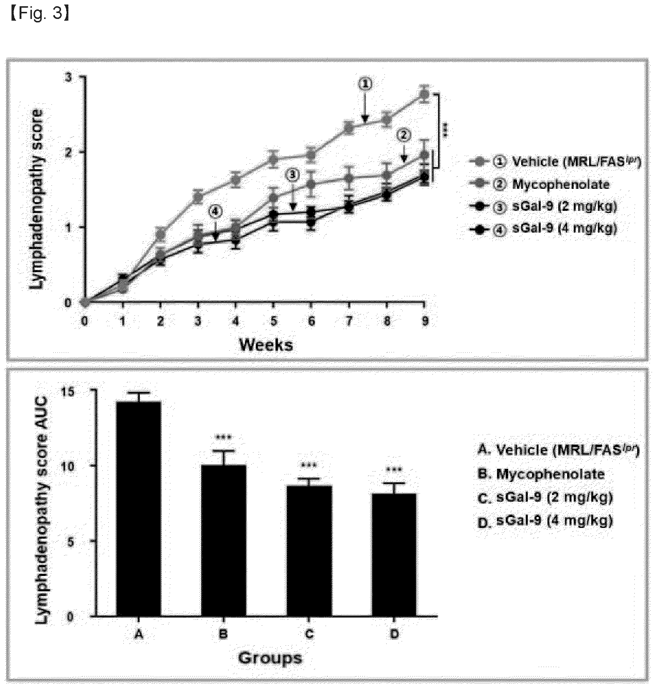

Furthermore, as shown in Fig. 3, it was confirmed that the lymphadenopathy score decreased in the positive control group (Mycophenolate) and the sGal-9 treatment groups (sGal-9 2 mg/kg and sGal-9 4 mg/kg) compared to the negative control group (Vehicle). Notably, the inhibitory effect on lymphadenopathy was more pronounced in the sGal-9 treatment groups (sGal-9 2 mg/kg and sGal-9 4 mg/kg) compared to the positive control group (Mycophenolate).

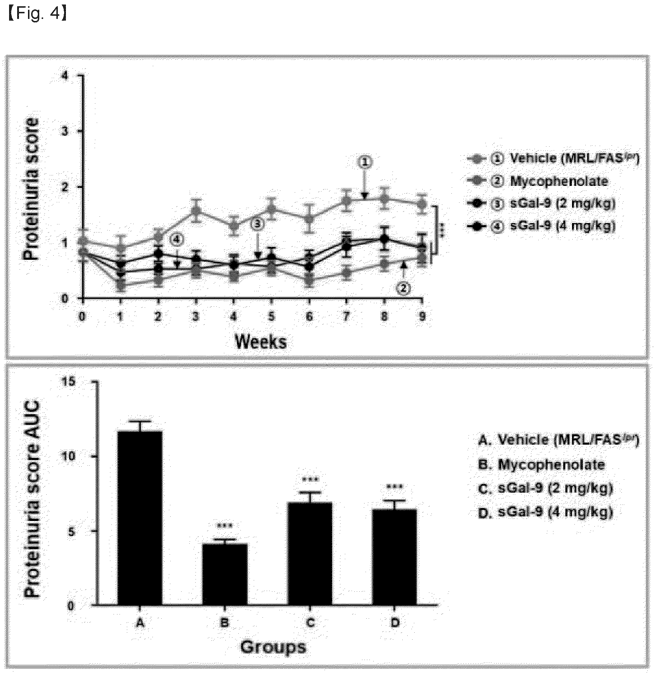

Furthermore, as shown in Fig. 4, it was confirmed that proteinuria decreased in the positive control group (Mycophenolate) and the sGal-9 treatment groups (sGal-9 2 mg/kg and sGal-9 4 mg/kg) compared to the negative control group (Vehicle).

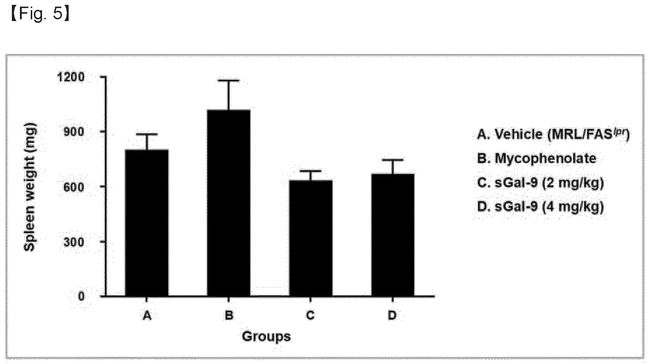

Additionally, as shown in Fig. 5, it was confirmed that spleen weight decreased in the sGal-9 2 mg/kg treatment group compared to the negative control group (Vehicle).

### <Experimental Example 3> Histomorphological Evaluation After Administration of sGal-9 in an In Vivo Model of Systemic Lupus Erythematosus

In order to evaluate the efficacy of the sGal-9 of the present invention in an *in vivo* model of systemic lupus erythematosus, hematoxylin and eosin (H&E) staining and IgG immunofluorescence staining were performed to examine histological changes in the renal tissue of the experimental animals of <1-1>.

Specifically, after the completion of the experiment, the experimental animals of <1-1> were sacrificed, and renal tissues were collected. The collected renal tissues were fixed and dehydrated in 10% formaldehyde and subsequently embedded in paraffin. The fixed renal tissues were sectioned to a thickness of 2 µm and stained with hematoxylin and eosin (H&E). The sectioned tissues were covered with a coverslip and observed under an optical microscope at 400× magnification. Four regions were imaged for each tissue, and two or more researchers assigned scores based on the evaluation items and criteria for histomorphological assessment shown in [Table 3]. The average of the researchers' scores was determined as the scoring value, and the mean of the scoring values for the four regions was calculated as the final score for each tissue.

**[Table 3]**

| Score | Glomerular Lesions |
|---|---|
| 0 | No recognizable lesion in glomeruli |
| 1 | Mild cell proliferation and/or cell infiltration |
| 2 | Same as score 1 with mesangial proliferation, lobulation and hyaline droplet, associated with macrophage infiltration |
| 3 | Same as score 2 with crescent and granuloma formation and/or hyalinosis |

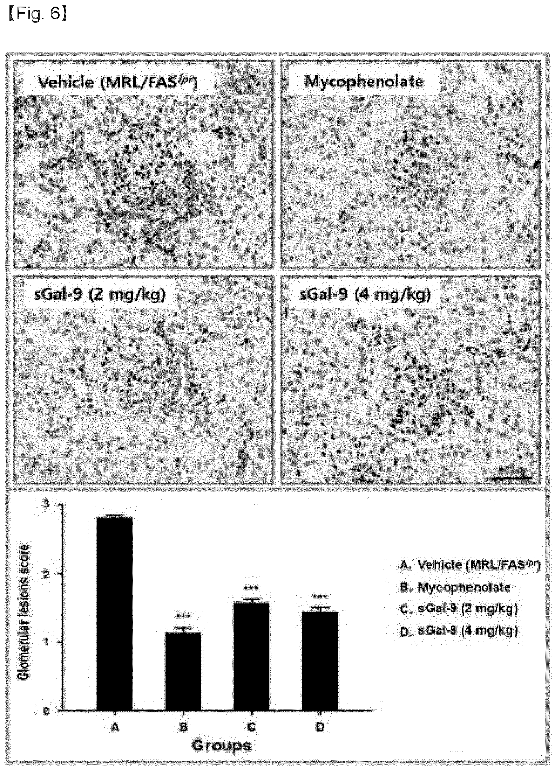

Additionally, the collected kidney tissues were frozen, sectioned at a thickness of 4 µm, and fixed in cold acetone for 5 minutes, followed by two washes with phosphate-buffered saline (PBS) for 5 minutes each. To eliminate non-specific reactions, blocking was performed for 30 minutes using a PBS solution containing 1% bovine serum albumin and 0.05% Tween-20. The sections were then incubated at room temperature for 1 hour with FITC-conjugated goat anti-mouse IgG antibody (1:200, AP308F, Merck Millipore). After three washes with PBS for 5 minutes each, nuclear staining was performed using DAPI, and the sections were covered with a cover glass using mounting medium. The samples were then examined using a confocal laser scanning microscope (C2 Plus, Nikon). As a result, as shown in Fig. 6, the negative control group (Vehicle) exhibited distinct signs of glomerulonephritis, including mesangial proliferation, infiltration of inflammatory cells within and around the glomeruli, and tubular destruction. In contrast, the positive control group (Mycophenolate) and the sGal-9 treatment groups (sGal-9 2 mg/kg and sGal-9 4 mg/kg) showed a reduction in inflammatory cell infiltration within the glomeruli. Furthermore, it was confirmed that the glomerular lesion scores decreased in the positive control group (Mycophenolate) and the sGal-9 treatment groups (sGal-9 2 mg/kg and sGal-9 4 mg/kg) compared to the negative control group (Vehicle).

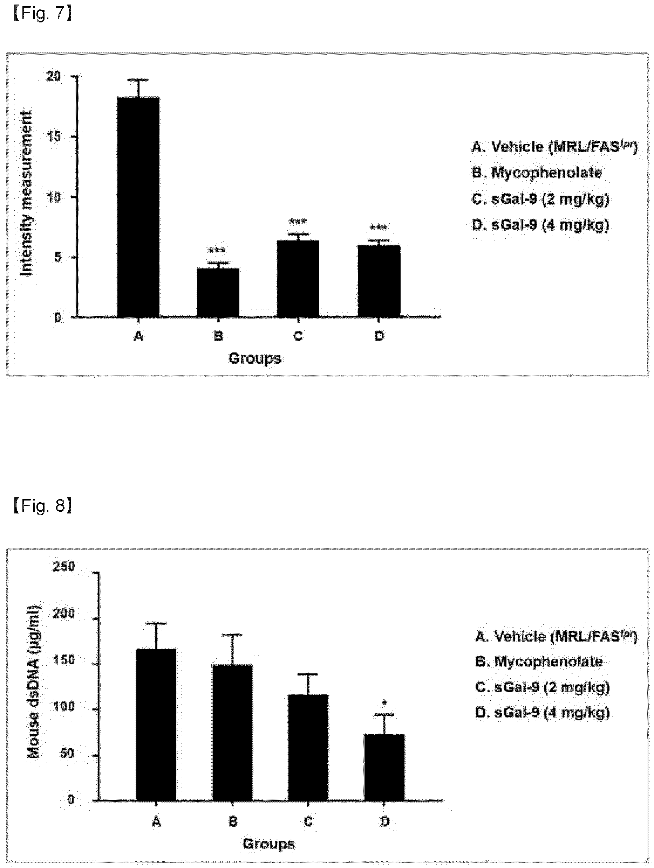

Furthermore, as shown in Fig. 7, it was confirmed that IgG deposition decreased in the positive control group (Mycophenolate) and the sGal-9 treatment groups (sGal-9 2 mg/kg and sGal-9 4 mg/kg) compared to the negative control group (Vehicle).

Based on the above results, it was confirmed that the sGal-9 of the present invention reduces inflammatory cell infiltration within and around the glomeruli as well as immune complex deposition in the kidneys, thereby exhibiting a therapeutic effect on lupus nephritis and glomerulonephritis.

### <Experimental Example 4> Analysis of Plasma Anti-dsDNA Antibody Levels After Administration of sGal-9 in an In Vivo Model of Systemic Lupus Erythematosus

Anti-dsDNA antibodies are a type of antinuclear antibody (ANA) that target intracellular nuclear components and are present at high titers in systemic lupus erythematosus (SLE). These antibodies are associated with the persistent progression of kidney inflammation and damage. Therefore, to evaluate the efficacy of the sGal-9 of the present invention in an *in vivo* model of systemic lupus erythematosus, the plasma concentration of anti-dsDNA antibodies was measured in the blood samples of the experimental animals from <1-1>.

Specifically, after the completion of the experiment, cardiac blood collection was performed on the experimental animals from <1-1 >. The collected blood was allowed to coagulate for 30 minutes using a capillary blood collection tube (Cat. 365967, BD) and then centrifuged at 6,000 × g for 10 minutes to obtain plasma. The obtained plasma was diluted at a ratio of 1:2,500, and the concentration of anti-dsDNA antibodies was measured using a Mouse Anti-dsDNA IgG Antibody ELISA Kit (Cat. 3031, Chondrex) according to the manufacturer's protocol.

As a result, as shown in Fig. 8, it was confirmed that the concentration of anti-dsDNA antibodies decreased in the positive control group (Mycophenolate) and the sGal-9 treatment groups (sGal-9 2 mg/kg and sGal-9 4 mg/kg) compared to the negative control group (Vehicle). Notably, the sGal-9 4 mg/kg treatment group (sGal-9 4 mg/kg) exhibited a superior reduction in anti-dsDNA antibody levels compared to the positive control group (Mycophenolate).

### [Industrial Applicability]

The recombinant stabilized Galectin-9 protein of the present invention has been confirmed to exhibit safety in a systemic lupus erythematosus (SLE) animal model, reduce skin lesions, lymphadenopathy, and proteinuria caused by lupus, ameliorate lupus nephritis and glomerulonephritis, and decrease the concentration of anti-dsDNA antibodies in plasma. Accordingly, the recombinant stabilized Galectin-9 protein of the present invention can be effectively used as an active ingredient in a composition for preventing or treating lupus or glomerulonephritis.

## Claims

1. A pharmaceutical composition for preventing or treating lupus or glomerulonephritis, comprising a recombinant stabilized Galectin-9 protein having the amino acid sequence set forth in Sequence No. 1 or a polynucleotide encoding the same as an active ingredient.

2. The pharmaceutical composition according to claim 1, wherein the recombinant stabilized Galectin-9 protein is further a protein in which the first amino acid residue from the N-terminus of the amino acid sequence set forth in Sequence No. 1 is deleted.

3. The pharmaceutical composition according to claim 1, wherein the lupus is selected from the group consisting of systemic lupus erythematosus (SLE), systemic lupus, discoid lupus, drug-induced lupus, neonatal lupus, and lupus nephritis.

4. The pharmaceutical composition according to claim 1, wherein the pharmaceutical composition reduces skin lesions, lymphadenopathy, or proteinuria caused by lupus.

5. The pharmaceutical composition according to claim 1, wherein the pharmaceutical composition reduces the concentration of anti-dsDNA antibodies in the blood.

6. The pharmaceutical composition according to claim 1, wherein the pharmaceutical composition further comprises a pharmaceutically acceptable carrier.

7. The pharmaceutical composition according to claim 1, wherein the pharmaceutical composition is formulated for oral administration, intramuscular administration, intravenous administration, intraperitoneal administration, subcutaneous administration, intradermal administration, or topical administration.

8. The pharmaceutical composition according to claim 1, wherein the pharmaceutical composition is formulated in a dosage form selected from the group consisting of tablets, capsules, injections, troches, powders, granules, solutions, suspensions, internal liquids, emulsions, syrups, suppositories, vaginal tablets, and pills.

9. A health functional food composition for preventing or ameliorating lupus or glomerulonephritis, comprising a recombinant stabilized Galectin-9 protein having the amino acid sequence set forth in Sequence No. 1 or a polynucleotide encoding the same as an active ingredient.

10. A method for preventing or treating lupus or glomerulonephritis, comprising administering to a subject a pharmaceutical composition comprising a recombinant stabilized Galectin-9 protein having the amino acid sequence set forth in Sequence No. 1 or a polynucleotide encoding the same.

11. Use of a recombinant stabilized Galectin-9 protein having the amino acid sequence set forth in Sequence No. 1 or a polynucleotide encoding the same for the manufacture of a pharmaceutical composition for preventing or treating lupus or glomerulonephritis.

12. Use of a recombinant stabilized Galectin-9 protein having the amino acid sequence set forth in Sequence No. 1 or a polynucleotide encoding the same for the manufacture of a health functional food composition for preventing or ameliorating lupus or glomerulonephritis.
